# DEMANDE DE BREVET EUROPEEN

(11) **EP 1 214 917 A1**
(43) Date de publication de la demande: **19.06.2002**
(21) Numéro de dépôt: 01870042.7
(22) Date de dépôt: 13.03.2001
(51) Int. Cl.: A61F 2/06

(54) **Endoprothèse luminale modulable**

(30) Priorité: 12.12.2000 BE 20000783
(71) Demandeur: Frid, Noureddine, 1650 Beersel (BE)
(72) Inventeur: Frid, Noureddine, 1650 Beersel (BE)
(74) Mandataire: Vandeberg, Marie-Paule L.G.

(57) **Abrégé**

Une endoprothèse luminale modulaire munie d'une armature, telle qu'un stent, et plus particulièrement une endoprothèse pour vaisseaux sanguins.
Cette endoprothèse est munie d'une armature tressée; l'armature (6) comprend une pluralité de couches (8, 10, 12) interconnectées entre elles, chacune de ces couches (8, 10, 12) étant formée de deux nappes de fils (14), respectivement dextrogyre et lévogyre entrelacées entre elles, formant une trame, une pluralité de fils (14) d'une couche déterminée (8, 10, 12) étant intégrés dans la trame d'au moins une des couches adjacentes (8, 10, 12).

## Description

L'invention concerne les endoprothèses luminales munies d'une armature, tels que les stents, et plus particulièrement les endoprothèses pour vaisseaux sanguins.

L'implantation d'endoprothèses luminales est devenue au fil des ans une technique approuvée pour traiter les anévrismes, l'athérosclérose etc.

Cependant, un problème crucial n'a toujours pas été résolu à ce jour : la parfaite adéquation des caractéristiques mécaniques de ces endoprothèses et des organes dans lesquels elles sont implantées.

Même si un soin tout particulier a été apporté pour répondre à ces critères au moment de l'implantation, il se produit invariablement, à la longue, une disparité. Le corps humain subit en effet des altérations dues au vieillissement, tandis que l'endoprothèse présente un problème de stabilité dans le temps : rupture des filaments, altération de la structure, augmentation éventuel du diamètre.

Les caractéristiques mécaniques d'une endoprothèse sont déterminées essentiellement par son armature. Quoiqu'il en existe de différents types, l'armature à ce jour la plus adéquate est l'armature tressée, telle que décrite notamment par Didcott dans GB-1205743, ou dans US 5 061 275.

Ce type d'armature se comprime aisément pour l'insertion, résiste bien à l'écrasement et conserve une souplesse relative compatible avec celle des vaisseaux sanguins ; la structure s'adapte au tracé sinueux d'artères rigides à traiter .

Jusqu'à présent, la recherche de l'armature optimale s'était orientée vers le choix du matériau, du pas de tressage, etc.

Ces recherches butent inévitablement sur une série de critères pratiques.

En adoptant un pas de tressage très faible ou en choisissant des fils épais, on accroît la force radiale (résistance à l'écrasement) mais on perd de la souplesse. En outre, un pas réduit signifie un taux de raccourcissement élevé au moment de la mise en place de l'endoprothèse, ce qui entraîne un manque de précision lors du largage.

A l'inverse, un pas élevé (et des fils minces) confèrent à l'armature une grande souplesse mais une faible tenue à l'écrasement.

Une nouvelle conception de l'armature permet de résoudre ces problèmes.

L'objet de l'invention est une endoprothèse munie d'une armature tressée caractérisée en ce que l'armature comprend une pluralité de couches interconnectées entre elles, chacune de ces couches étant formée de deux nappes de fils, respectivement dextrogyre et lévogyre entrelacées entre elles, formant une trame, une pluralité de fils d'une couche déterminée étant intégrés dans la trame d'au moins une des couches adjacentes.

Les fils de l'armature peuvent être des fils métalliques ou des fils en matériau synthétique, biocompatibles.

Dans le cas d'une armature métallique, les fils sont de préférence choisis parmi les matériaux suivants [acier inoxydable, Phynox, Elgyloy, titane et alliages ou titane Nitinol].

Dans le cas d'une armature en matériaux synthétiques, ces matériaux peuvent être ou non assimilables par l'organisme.
Suivant une forme de réalisation avantageuse, l'armature comprend des fils d'épaisseurs différentes.

L'épaisseur des fils peut s'étager sur une gamme commençant à partir de 25 microns.

L'avantage de l'utilisation de fils minces est qu'ils perturbent peu le flux sanguin : il est donc possible de les utiliser pour des stents sans couches de revêtement, qui assurent la filtration de particules susceptibles de provoquer des thromboses voire des attaques cérébrales.

L'avantage de l'utilisation de fils plus épais est qu'ils assurent une meilleure tenue contre la paroi des vaisseaux et permettent à l'endoprothèse d'encaisser sans dommages les diverses sollicitations auxquels les vaisseaux sont soumis, notamment au niveau du cou et du genou.

L'utilisation de couches interconnectées résout également deux problèmes épineux connus de la technique antérieure : en utilisant des nappes de fils de caractéristiques mécaniques différentes, on constate que l'endoprothèse classique aura tendance à migrer longitudinalement.

A l'inverse, dans une prothèse suivant l'invention, les propriétés mécaniques des fils des différentes nappes peuvent être équilibrées, de façon à se compenser parfaitement.

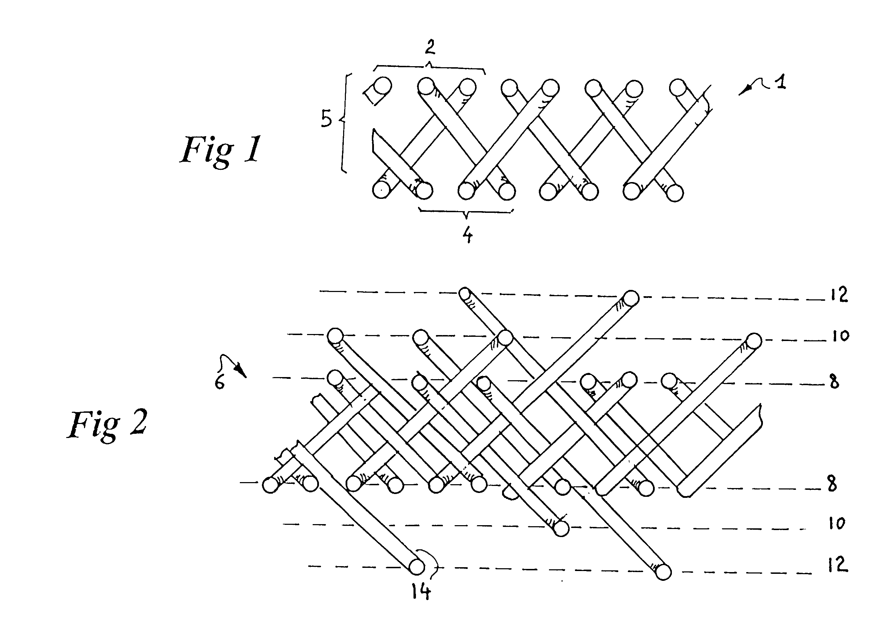

D'autres particularités et avantages de l'invention ressortiront de la description ci-après de modes de réalisation particuliers de l'invention, référence étant faite aux dessins annexés dans lesquels
La Fig. 1 est une vue latérale d'une armature de stent tressé classique;
La Fig. 2 est une vue schématique simplifiée de l'armature de stent de l'invention.

L'armature tressée classique 1 est formée d'une tresse simple, deux nappes 2, 4 de fils, respectivement dextrogyre 2 et lévogyre 4 s'entrecroisent, formant une trame 2, 4 simple.

L'armature 6 de l'invention est une tresse multiple qui comprend, dans l'exemple illustré, trois couches 8, 10, 12 dont les nappes ne sont pas distinctes : au moment du tressage, un certain nombre de fils 14 des nappes de la première couche 8 sont entrelacés avec les nappes de la deuxième couche 10 et/ou de la troisième couche 12, formant une trame complexe( ceci vaut pour la figure représentée, mais il va de soi que l'entrelacement peut se poursuivre jusqu'à une Nième couche si le nombre de couches est N). Cette façon de procéder ouvre des possibilités énormes pour ajuster les caractéristiques de l'armature. Non seulement elle rend possible une grande variété de "standards" en fonction des organes visés, mais encore, elle permet pratiquement un ajustement cas par cas en jouant sur le pas du tressage, le diamètre et la nature des fils 14, la densité du tressage, le nombre des couches 8, 10, 12 et le nombre de fils 14 de différents diamètres et l'entrelacement des couches.

Il serait inutile d'évoquer tous ces avantages si les endoprothèses munies d'une telle armature ne pouvaient pas être mises en place avec les équipements existants.

Or, c'est un des aspects inattendus de l'invention, on constate qu'en dépit du grand nombre de fils 14 utilisés, de l'épaisseur des couches 8, 10, 12 successives, la complexité de la structure, la tresse multiple peut être très aisément réduite jusqu'à un diamètre comparable à celui d'une armature classique 1.

Il est donc possible d'utiliser sans difficulté un introducteur classique pour la mise en place d'une endoprothèse équipée de la nouvelle structure, même dans des vaisseaux de diamètre réduit.

Par ailleurs, l'endoprothèse de l'invention peut assumer de très grands diamètres (notamment pour l'οesophage) sans risque d'écrasement.

La présente structure permet également aux couches 8, 10, 12 constituées de fils 14 de différents diamètres d'agir en synergie. Des essais pratiques menés par des chirurgiens où l'on se contente d'introduire l'un dans l'autre deux stents 1 de caractéristiques différentes se sont soldées par des résultats mitigés ou des échecs, alors que la présente structure 6 accroît notablement la résistance à l'écrasement sans réduire la flexibilité.

Cette caractéristique est importante notamment dans le cas de traitement d'anévrismes. En effet, un anévrisme montre au fil du temps une tendance à se raccourcir dans le sens longitudinal. Un stent classique placé dans ces circonstances aura tendance à onduler et finalement à s'écraser, ce qui n'est pas le cas de la présente structure.

Comme il a été précisé plus haut, la structure en couche multiple 6 permet l'utilisation de fils de très fin diamètre (jouant le rôle d'une structure filtrante) en combinaison avec des fils de renfort plus épais.

L'entrelacement de ces fils assure une répartition régulière dans l'espace, assurant un maillage régulier propice à un filtrage uniforme des particules.

Outre leurs caractéristiques mécaniques propres, on peut également utiliser avec avantages les particularités confiées aux fils par un traitement technique adéquat.

On peut aussi tirer parti de l'usage de fils en Nitinol tels que décrits dans la demande PCT/BE 98/00076 pour obtenir un raffermissement de la structure après sa mise en place.

## Revendications

1. Endoprothèse luminale munie d'une armature tressée **caractérisée en ce que** l'armature (6) comprend une pluralité de couches (8, 10, 12) interconnectées entre elles, chacune de ces couches (8, 10, 12) étant formée de deux nappes de fils 14, respectivement dextrogyre et lévogyre entrelacées entre elles, formant une trame (5), une pluralité de fils (14) d'une couche déterminée (8, 10, 12) étant intégrés dans la trame d'au moins une des couches adjacentes (8, 10, 12).

2. Endoprothèse luminale suivant la revendication 1 **caractérisée en ce que** les fils (14) de l'armature (6) sont des fils biocompatibles métalliques ou des fils biocompatibles en matériau synthétique.

3. Endoprothèse luminale suivant la revendication 2 **caractérisée en ce que** le matériau des fils (14) est choisi parmi les matériaux suivants [acier inoxydable, Phynox, Elgyloy, titane et ses alliages, Nitinol].

4. Endoprothèse luminale suivant la revendication 1 **caractérisée en ce que** le matériau des fils (14) est synthétique, ce matériau pouvant être ou non assimilable par l'organisme.

5. Endoprothèse luminale suivant l'une quelconque des revendications précédentes **caractérisée en ce que** l'armature (6) comprend des fils (14) d'épaisseurs différentes.

6. Endoprothèse luminale suivant la revendication 5 **caractérisée en ce que** l'armature (6) comprend des couches formées de fils (14) d'épaisseur différente.

7. Endoprothèse luminale suivant l'une quelconque des revendications précédentes **caractérisée en ce que** l'armature comprend des fils (14) de natures différentes.

8. Endoprothèse luminale suivant l'une quelconque des revendications 5, 6 et 7 **caractérisée en ce que** l'épaisseur des fils (14) s'étage sur une gamme commençant à 25 microns.

9. Endoprothèse luminale suivant l'une quelconque des revendications précédentes **caractérisée en ce que** l'armature (6) est au moins partiellement dépourvue de couche de revêtement

10. Endoprothèse luminale suivant la revendication 9 précédentes **caractérisée en ce que** l'armature (6) comprend une pluralité de couches (8, 10, 12) formées essentiellement de fils (14) minces qui assurent la filtration de particules susceptibles de provoquer des thromboses.
